# EUROPEAN PATENT SPECIFICATION

(11) **EP 3 593 850 B1**
(45) Date of publication and mention of the grant of the patent: **28.09.2022**
(21) Application number: 19195053.4
(22) Date of filing: 13.03.2013
(51) Int. Cl.: A61M 25/00

(54) **DRY TO THE TOUCH VAPOR HYDRATION SLEEVE**
DAMPFHYDRATIERENDE HÜLLE MIT TROCKENGRIFF
MANCHON HYDRATANT PAR VAPEUR À TOUCHE SÈCHE

(30) Priority: 12.11.2012 US 201261725282 P
(43) Date of publication of application: 15.01.2020
(62) Divisional of application: 13716499.2
(73) Proprietor: Hollister Incorporated, Libertyville IL 60048 (US)
(72) Inventor: ROSTAMI, Shamsedin, Boars Hill, Oxfordshire OX1 5DR (GB); FITZPATRICK, James J., Co. Mayo (IE); CARTER, Enda F., Co. Mayo (IE); MURRAY, Michael G., Co. Mayo (IE); O'FLYNN, Padraig M., Co. Galway (IE); HENRY, Jerome A., Co. Mayo (IE); MORAN, Martin T., Co. Galway (IE); FOLEY, Adam J., Co. Mayo (IE)
(74) Representative: FRKelly

(56) References cited:
- EP-A2- 1 498 151
- WO-A2-2005/014055
- WO-A2-2007/146820
- US-A- 5 328 848

## Description

### RELATED APPLICATION

This application claims the benefit of and priority to U.S. Provisional Patent Application Serial No. 61/725,282, filed November 12, 2012.

### FIELD OF THE DISCLOSURE

The present disclosure is generally directed to a catheter assembly having a catheter shaft with a hydrophilic outer surface and, more particularly, to a catheter assembly having a protective sleeve that provides a barrier through which the catheter may be gripped/manipulated for inserting the catheter through the urethra and wherein, at point of use, the protective sleeve is dry to the touch.

### BACKGROUND OF THE DISCLOSURE

Catheter assemblies are a good option for many users who suffer from various abnormalities of the urinary system. A common situation is where single use, individually packaged, sterile ready-to-use catheters are utilized. An important criterion for single use, ready-to-use products is that they be entirely user-friendly upon removal from the packaging

It is quite common for single use, ready-to-use catheters to be provided with a surface treatment which uses a lubricant adapted to reduce friction in order to allow for easier and less traumatic catheter insertion and withdrawal. Currently, there are two major categories of catheters having lubricated surfaces, i.e., catheters having a gel lubricant (typically a water based lubricant) applied to the catheter shaft and catheters having a hydrated hydrophilic outer surface on the catheter shaft.

In a hydrophilic lubricated catheter, the catheter is typically provided with a thin hydrophilic coating adhered to the outer surface of the catheter shaft. When this hydrophilic coating is activated by a swelling medium, it provides a low coefficient-of-friction surface to facilitate catheter insertion and withdrawal. Hydrophilic lubricated catheters are activated when a hydrating agent such as liquid water or water vapor comes into direct contact with the hydrophilic coating on the catheter shaft.

In another form, the catheter can be made from a hydrophilic material in which case there need be no coating on the outer surface of the catheter shaft. Instead, the outer surface of the catheter itself is a hydrophilic material, and it provides the low coefficient-of-friction surface to facilitate catheter insertion. As with a hydrophilic coated catheter, a catheter made from a hydrophilic polymer material is activated when a hydrating agent directly contacts the outer surface.

When a catheter is removed from the package for insertion into the urethra, there are some disadvantages encountered. First, when the proximal insertion end of the catheter is introduced into the urethra it may pick up microorganisms that are likely to be prevalent in the distal portion of the urethra. These microorganisms are often carried by the proximal insertion end of the catheter into the bladder as it is fully inserted, thereby increasing the risk of infection. Second, the handling of the catheter by the user may also introduce microorganisms onto the surface of the catheter which can cause infection after catheter insertion. For hydrophilic lubricated catheters, these issues should be solved without interfering with activation of the hydrophilic outer surface.

Specifically, for a hydrophilic lubricated catheter, any attempt to: i) prevent pathogens from being picked up by the proximal insertion end of the catheter upon introduction into the distal portion of the urethra, and ii) prevent the introduction of microorganisms onto the surface of the catheter as a result of handling by the user, should be addressed in a manner that does not interfere with the hydrating agent coming into direct contact with the hydrophilic outer surface.

For hydrophilic lubricated catheters, sleeves covering the catheter shaft have generally not been available because the sleeve interferes with the flow of liquid water to the catheter surface that is required for activation by direct liquid contact. To overcome this problem, a recent alternative provides a vapor atmosphere inside the catheter package and forms the sleeve of a liquid impermeable, vapor permeable material so the vapor can reach the hydrophilic outer surface of the catheter. While this approach has proved to be quite successful, one drawback is that, as the vapor penetrates the sleeve, it leaves moisture on the outer surface of the sleeve which must be gripped by the user for advancement of the catheter through the urethra and into the bladder. WO 2007/146820 discloses a catheter product package and method of forming same in which the package comprises a sheet material wrapped about the catheter product to form a package for the catheter product. The catheter product extends generally longitudinally within the package, and the sheet material extends from a point beyond the proximal end to a point beyond the distal end of the catheter product. EP 1498151 discloses a urinary catheter pre-lubricated with a gel lubricant and a package assembly comprising a package defining an elongate sealed catheter receiving chamber carrying the urinary catheter with a guide arrangement within the chamber. WO 2005/014055 discloses a packaged catheter assembly and method of manufacturing and distributing the same. The assembly includes a gas impermeable package housing a catheter with a catheter receiving cavity.

### SUMMARY OF THE DISCLOSURE

A hydrophilic catheter that is fully hydrated in its packaging and delivered in a manner that is dry to the user's touch is achieved by various embodiments of the present disclosure. In each embodiment, an intermittent catheter having a hydrophilic coating is provided in a sleeve including opposing walls of a water impermeable material. Water impermeable polymers that are commonly known in the industry include homo- or copolymers of polycholorotrifluoroethylene, polyvinylidene chloride, polyolefins, polyethyleneterephthalate, polyethylenenaphthalenedicarboxylate, or coated or metalized or metal oxide coated polymers which improve their barrier performance to water vapor permeation. Lists of other water impermeable polymers can be found in the literature such as "Permeability Properties of Plastics and Elastomers", Second Edition: A Guide to Packaging and Barrier Materials, by L.K. Massey. Also in Polymer Handbook, 3rd Ed, pages VI/437 to VI/445, by J. Brandrp and E.H. Immergut.

Examples of such sleeve materials include SARANEX^{®} barrier films, which are liquid and water vapor impermeable multilayer films available from The Dow Chemical Company. The opposing walls may be formed as two separate sheets of such sleeve films ultimately sealed to one another along all sides, or preferably as a single sheet of the sleeve film that is folded over and ultimately sealed on three sides.

A membrane (formed from what is known as a breathable polymer film, i.e., water vapor permeable material, such as micro porous polyethylene films - commonly made by addition of calcium carbonate particles to polyethylene. The film is made from the mixture and stretched to produce a porous film with high permeability to water vapor- copolyurethane, or copolyester films) is sealed to an inner side of at least one of the sleeve sheets (i.e., on the side of at least one of the sleeve sheets facing the catheter), and, after vapor phase water permeates through the membrane, the region of the sleeve containing the catheter eventually contains a sufficient amount of water vapor to activate the hydrophilic coating on the catheter, the liquid phase water having been introduced to the region defined between the water vapor permeable membrane and the wall of the water impermeable sleeve to which the water vapor permeable material is sealed. In other words, initially, the water is isolated from the catheter. However, over a period of time (the length of which depends upon the permeability of the water vapor permeable material, among other factors), at least some of the water changes from a liquid to a vapor phase, migrates across the water vapor permeable material, and humidifies the region between the two water impermeable sleeves in which the catheter is disposed, thereby contacting and activating the hydrophilic coating. As used herein, a breathable polymer film or a water vapor permeable material refers to a membrane having a water vapor permeability (moisture vapor transmission rate) greater than 300 g/m²/day, greater than 500 g/m²/day, greater than 1000 g/m²/day, greater than 2000 g/m²/day or preferably greater than 3000 g/m²/day, as measured according to ASTM E-96 Procedure E - Desiccant Method at 100°F (37.8°C) and 75% Relative Humidity.

In certain embodiments of the present disclosure, a strip of fabric is provided between the inner side of at least one of the sleeve sheets to which the water vapor permeable material is sealed. The fabric strip soaks in the liquid water, avoiding sloshing within the sleeve. The water-soaked fabric strip also helps to distribute water across the entire length of at least the hydrophilic-coated region of the catheter, by means of wicking or capillary action. The fabric strip also serves to conceal the water from view through the sleeve, particularly when the sleeve walls are made of a transparent or translucent material.

In certain additional embodiments of the present disclosure, the water-soaked strip of fabric or similar source of liquid phase water is embedded within a water vapor permeable sheath that is provided in the sleeve and extends substantially the length of the hydrophilic-coated region of the catheter while the catheter is disposed within the package.

In other embodiments of the present disclosure, no fabric strip is provided, and the water in liquid phase is loose within the region between the inner side of at least one of the sleeve sheets to which the water vapor permeable material is sealed, and the water vapor permeable material, or alternately, the liquid phase water is provided within one or more bladders of a liquid impermeable, water vapor permeable sheath provided in the sleeve and extending substantially the length of the hydrophilic-coated region of the catheter while the catheter is disposed within the package. To minimize sloshing, the region in which the liquid-phase water is provided may be supplied with a volume of water that completely fills that region.

An introducer tip may be provided at a first end of the sleeve, adjacent an eyed tip end of the catheter. The catheter may be removed from the sleeve for use by maneuvering the catheter through the sleeve, and beginning with the eyed tip end, urging the catheter through the introducer tip. A drainage opening may be provided at an end of the sleeve opposite from the introducer tip by tearing off a sealed end portion of the sleeve adjacent a funnel end of the catheter. A notch may be provided in at least one side of the sealed end portion of the sleeve to facilitate initiation of the tear. Since the funnel end of the catheter does not fit through the introducer tip, the funnel serves as a stop, maintaining communication between the catheter and the sleeve, and the sleeve may be used to direct flow of urine into a collection container or a toilet. Alternately, the end of the sleeve adjacent the funnel when the sleeve is fully extended over the catheter may be sealed, wholly or partially, to a neck at the base of the funnel (i.e. at the smaller-diameter end of the funnel in which the tube of the catheter is received). In one aspect, a dry-to-the touch, ready- to-use catheter assembly is provided. The catheter assembly includes a catheter having an insertable portion with a hydrophilic outer surface in an activated condition. A liquid and vapor impermeable sleeve covers at least the insertable portion of the catheter. An amount of liquid is disposed between the sleeve and the insertable portion of the catheter wherein the amount of liquid is contained by a liquid flow interfering element that substantially interferes with liquid flow. A vapor atmosphere within the liquid and vapor impermeable sleeve wherein the vapor atmosphere is produced by vapor donated from the amount of liquid contained by the liquid flow interfering element. The liquid flow interfering element is disposed between the sleeve and the insertable portion of the catheter such that the liquid flow interfering element substantially prevents direct liquid contact between the amount of liquid contained by the liquid flow interfering element and the hydrophilic outer surface of the insertable portion of the catheter while permitting sufficient direct vapor contact to place the hydrophilic outer surface in the activated condition.

In another aspect, a dry-to-the touch, ready-to-use hydrophilic intermittent catheter assembly includes a catheter having an insertable portion with a hydrophilic outer surface in an activated condition. A liquid and vapor impermeable sleeve covers at least the insertable portion of the catheter. An amount of liquid is disposed between the sleeve and the insertable portion of the catheter. The amount of liquid is contained within a liquid flow interfering element that substantially interferes with liquid flow. A vapor atmosphere present within the liquid and vapor impermeable sleeve is produced by vapor donated from the amount of liquid contained within the liquid flow interfering element. The liquid flow interfering element is formed of a material and disposed between the sleeve and the insertable portion of the catheter in a manner that substantially prevents direct liquid contact between the amount of liquid contained by the liquid flow interfering element and the hydrophilic outer surface of the catheter while permitting sufficient direct vapor contact to place the hydrophilic outer surface in the activated condition. Additionally, the liquid flow interfering element includes a liquid impermeable, vapor permeable membrane sealed to an inner surface of the sleeve with the liquid being confined between the membrane and the inner surface of the sleeve. The sleeve is sealed in a manner defining a closed cavity containing at least the insertable portion of the catheter, the liquid flow interfering element, and the amount of liquid contained within the liquid flow interfering element.

In a further aspect, a dry-to-the touch, ready-to-use hydrophilic intermittent catheter assembly includes a catheter having an insertable portion with a hydrophilic outer surface in an activated condition. The catheter includes an eyed inlet end and a funnel at an opposite end. A liquid water impermeable, water vapor permeable inner sleeve covers the insertable portion of the catheter and is sealed to a collar of the funnel. A liquid water impermeable and water vapor impermeable outer sleeve encloses at least the inner sleeve and the insertable portion of the catheter and an amount of liquid water is disposed between an exterior of the inner sleeve and an interior of the outer sleeve. A vapor atmosphere present within the inner sleeve is produced by vapor donated by the amount of liquid. The inner sleeve substantially prevents direct liquid contact between the amount of liquid disposed between the inner and outer sleeves while permitting sufficient direct vapor contact to place the hydrophilic outer surface in the activated condition.

### BRIEF DESCRIPTION OF THE SEVERAL VIEWS OF THE DRAWING

Fig. 1 is a perspective view, broken away, of a catheter in a sleeve assembly of a first embodiment of the present disclosure;
Fig. 2 is a cross-sectional view taken along lines 2-2 of Fig. 1;
Fig. 3 is a perspective view, broken away, of a catheter in a sleeve assembly of a second embodiment of the present disclosure;
Fig. 4 is a cross-sectional view taken along lines 4-4 of Fig. 3;
Fig. 4A is a top plan view of another embodiment of a catheter and sleeve assembly;
Fig. 4B is a cross-sectional view of the catheter and sleeve assembly of Fig. 4A;
Fig. 5 is a cross-sectional view of a catheter in a packaged sleeve assembly of a third embodiment of the present disclosure;
Fig. 6 is a top plan view of the catheter and sleeve assembly of Fig. 5, with a top face of packaging material removed for clarity, illustrating the catheter and sleeve substantially withdrawn from the package and a wetted water-containing liquid impermeable, vapor permeable membrane, which is tethered to the package, left behind in the package;
Fig. 7 is a cross-sectional view taken along lines 7-7 of Fig. 5;
Fig. 8 is a side plan view, partially cut away, of a catheter in a sleeve assembly according to the embodiments of Figs. 1 and 2;
Fig. 9 is a front plan view, partially cut away, of a catheter in a sleeve assembly according to the embodiments of Figs. 1 and 2;
Fig. 10 is a side plan view, partially cut away, of a catheter in a sleeve assembly according to a fourth embodiment, in which the sleeve is provided with at least one wetted water-containing liquid impermeable, vapor permeable membrane, the sleeve terminates at a neck end of the funnel of the catheter, and the sleeve walls are sealed to the neck of the funnel;
Fig. 11 is a front plan view, partially cut away, of the catheter in a sleeve assembly according to the embodiment of Fig. 10;
Fig. 12 is a side elevational view, partially in section, illustrating the catheter being delivered through an introducer tip, with the sleeve crumpled, and wherein the sleeve of the embodiment of Figs. 10 and 11 is sealed to the neck of the funnel of the catheter;
Fig. 13 is a side plan view, partially cut away, of a catheter in a sleeve assembly according to a fifth embodiment, in which the sleeve is provided with at least one liquid water-containing region separated from the catheter by a liquid water impermeable, water vapor permeable membrane, and the sleeve extends past the funnel end of the catheter and not sealed at that end;
Fig. 14 is a front plan view, partially cut away, of the catheter in a sleeve assembly according to the embodiment of Fig. 13;
Fig. 15 is a side elevational view, partially in section, illustrating the catheter being delivered through an introducer tip, and with the sleeve crumpled, and wherein the sleeve of the embodiment of Figs. 13 and 14 is not sealed beyond the funnel of the catheter;
Fig. 16 is a side elevational view illustrating a funnel end of the catheter engaged with an opening at the introducer tip end of the sleeve, preventing the funnel from falling out of the sleeve, and with the sleeve pulled back to direct flow of urine;
Fig. 17 illustrates the sleeve directing flow of urine toward a collection container;
Fig. 18 is a side elevational view illustrating a sixth embodiment of a packaged catheter of the present disclosure wherein a catheter is disposed in a liquid impermeable, vapor permeable sleeve having two polymer films sealed to the collar of a funnel of the catheter, where both the catheter and the sleeve are contained in a liquid and vapor impermeable barrier film that is also sealed to the collar of the funnel, with the funnel exposed proudly of both the sleeve and the barrier film;
Fig. 19 is a front plan view of the packaged catheter of Fig. 18;
Fig. 20 is a side elevational view illustrating a seventh embodiment of a packaged catheter of the present disclosure wherein a catheter is disposed in a liquid impermeable, vapor permeable sleeve having two polymer films sealed to the edge or collar end of a funnel of the catheter, where both the catheter and the sleeve are contained in a liquid and vapor impermeable barrier film sealed about an end of the funnel opposite the collar end, with the funnel exposed proudly of the sleeve, and an end of the funnel opposite the collar end exposed from the barrier film;
Fig. 21 is a front plan view of the packaged catheter of Fig. 20;

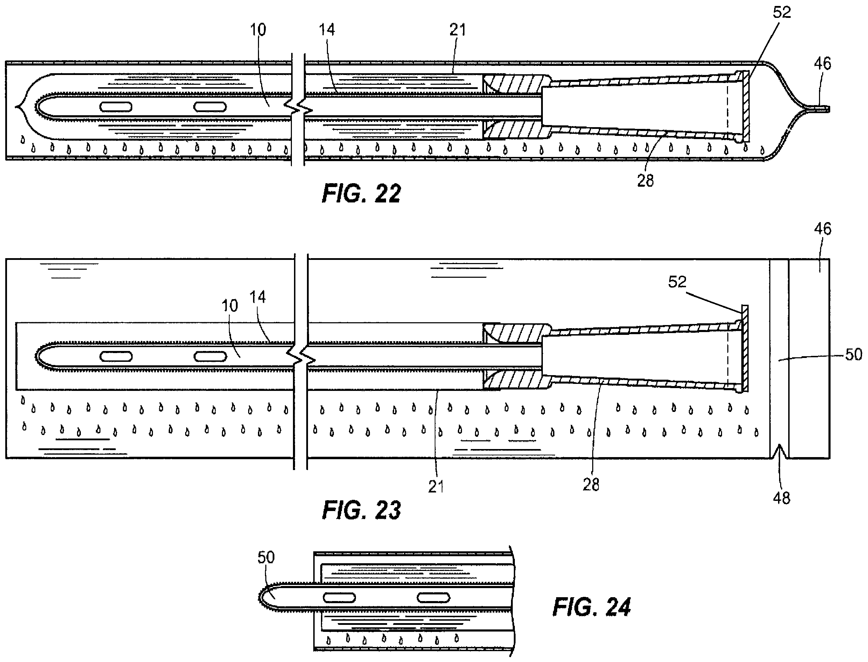

Fig. 22 is a side elevational view illustrating an eighth embodiment of a packaged catheter of the present disclosure wherein a catheter is disposed in a liquid impermeable, vapor permeable sleeve having two polymer films sealed to the edge or collar end of a funnel of the catheter, where both the catheter and the sleeve are contained in a liquid and vapor impermeable barrier film, the walls of the barrier film being sealed to one another beyond the funnel of the catheter thereby enclosing the entirety of the funnel within the barrier film, and the barrier film including a tearable track permitting controlled opening of the barrier film to facilitate drainage of water therefrom;
Fig. 23 is a front plan view of the packaged catheter of Fig. 22; and
Fig. 24 is a front plan view of a portion of the catheter protruding through both the water permeable membranes 20 and the water impermeable barrier 12.

### DETAILED DESCRIPTION OF THE PREFERRED EMBODIMENTS

A catheter 10, such as an intermittent urinary catheter, is provided within a sleeve 12 of a liquid water and water vapor impermeable film, such as SARANEX^{®} barrier film available from The Dow Chemical Company. The sleeve may be comprised of an elongate tubular film, or may include sleeve walls 16, 18 formed either by a single sheet of film folded over onto itself and sealed along the overlapping longitudinal end, or two distinct sheets of film bonded to one another. At least an insertable portion of the catheter 10, i.e. that portion of the catheter 10 which is insertable into a urethra of a patient to facilitate the drainage of urine from a patient's bladder, is provided with a hydrophilic coating 14. The first and second sleeve walls 16, 18 may be independent sheets of barrier film ultimately sealed to one another along all sides, or may be a single sheet of barrier film folded onto itself and ultimately sealed to itself along its perimeter. An intermediate liquid water impermeable, water vapor permeable membrane 20 is sealed to an inner surface of at least one of the first and second sleeve walls 16, 18 to form a region or pocket 23 therebetween. The liquid water impermeable, water vapor permeable membrane 20 may be made, for example, of PU copolymers, such as those offered by Mylan Technologies, Smith & Nephew, Bayer, or Lubrizol; stretched CaCO 3-filled polyethylene (or polypropylene) film, available from RKW or Tredegar, or copolyesters offered by RKW under the trade name of APTRA^{®} M, or any similar products with greater than 300 g/ m²/day, greater than 500 g/ m²/day, greater than 1000 g/ m²/day, greater than 2000 g/ m²/day or preferably greater than 3000 g/ m²/day, as measured according to ASTM E-96 Procedure E - Desiccant Method at 100°F (37.8°C) and 75% Relative Humidity, including thin films made of at least one of the group of copolyurethanes, copolyesters, calcium carbonate-filled polyethylene, and calcium carbonate-filled polypropylene, and preferably extends at least as long as the length of the hydrophilic coated portion of the catheter 10.

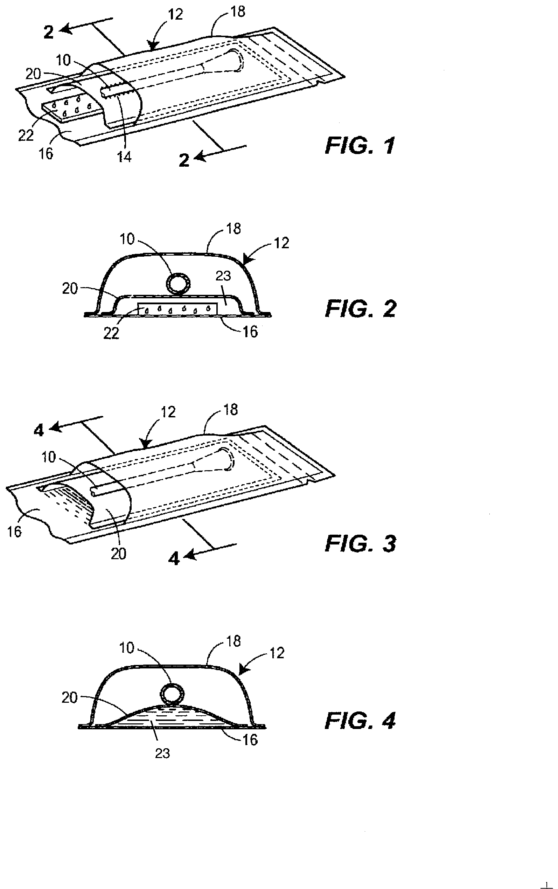

In a first embodiment, illustrated in Figs. 1 and 2, a wicking element 22 is provided in the region 23 between the liquid water impermeable, water vapor permeable membrane 20 and the inner surface of the at least one of the first and second sleeve walls 16, 18 to which the water vapor permeable membrane 20 is sealed (i.e., the inner surface of the sleeve 12). Water in liquid phase is added to the wicking element 22, which evenly disperses liquid confined between the water vapor permeable membrane 20 and the inner surface of the sleeve 12. The catheter 10 is provided in a region of the sleeve 12 that is on an opposite side of the water vapor permeable membrane 20 from the wicking element 22.

In this first embodiment, the water vapor permeable membrane 20, together with the wicking element 22, serve as a liquid flow interfering element disposed between the sleeve 12 and the insertable portion of the catheter 10 in a manner that prevents sufficient direct liquid contact with the hydrophilic coating on the catheter 10 to place the hydrophilic outer surface of the catheter 10 in an activated condition. Due to the liquid and vapor impermeability of the sleeve 12 and the vapor permeability of the liquid impermeable, water vapor permeable membrane 20, as the liquid water changes phase from liquid to vapor, the liquid flow interfering element permits water vapor to permeate across the water vapor permeable membrane 20, eventually achieving sufficient direct vapor contact with the insertable length of the catheter 10 to place the hydrophilic outer surface in the activated condition. After such sufficient direct vapor contact has placed the hydrophilic outer surface in the activated condition, the catheter assembly is ready for patient use. As used herein, the term "ready-to-use" refers to a catheter assembly having a hydrophilic outer surface in the activated condition such that the catheter 10 is ready for insertion into a patient's urethra without the need for further lubrication of the catheter 10.

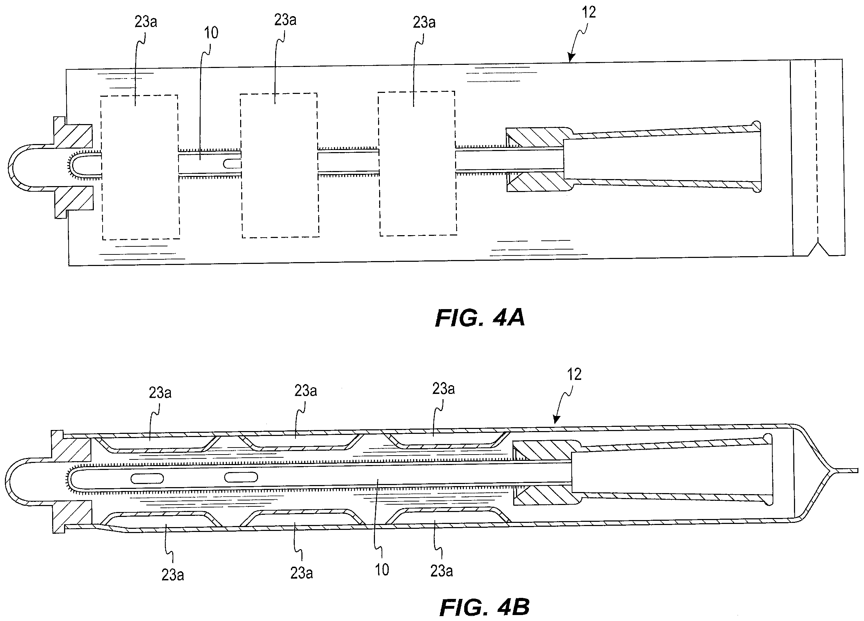

In a second embodiment, illustrated in Figs. 3 and 4, no such wicking element is provided, but an amount of water in liquid phase is provided within the region 23 between the liquid water impermeable, water vapor permeable membrane 20 and the inner surface of sleeve wall 16. In this second embodiment, the liquid water impermeable, water vapor permeable membrane 20 serves as a liquid flow interfering element. The region 23 in which the liquid-phase water is provided may be supplied with a volume of water that completely or partially fills that region 23.

As illustrated in Figs. 4A and 4B, to prevent pooling toward one end of the region 23 or potential uneven distribution of water vapor along the hydrophilic coating or hydrophilic surface of the catheter, the region 23 may be divided between a plurality of pockets or sachets 23a spaced along the length of catheter 10 and/or the length of sleeve 12. Each of the sachets 23 a defines a bladder containing liquid-phase water. In the embodiment illustrated in Figs. 4A and 4B, each sachet 23a is formed from a sheet of water vapor permeable membrane 20 sealed to one of sleeve walls 16, 18. In other embodiments, sleeve walls 16, 18 do not form part of the sachets 23a and the sachets 23a are made from materials that are discrete from the sleeve walls 16, 18. In such an embodiment, the sachets 23a may have top and bottom walls formed from a water vapor permeable membrane or may include one wall made of a water vapor permeable membrane and another wall made from a different material, which is not necessary water vapor permeable. When the sachets 23 a are discrete from the sleeve walls 16, 18, the sachets may be inserted into the sleeve and against walls 16, 18 prior to sealing of the sleeve.

Additionally, in the illustrated embodiment, there are a total of six sachets 23 a - three above the catheter 10 and three below the catheter 10. In other embodiments, there may be more or less than six sachets 23 a and the sachets may be arranged only above or below the catheter 10 or may be arranged in an alternating arrangement wherein the sachets 23a alternate between being above and below the catheter 10. In still other embodiments, the sachets may be attached to form groups of sachets or may be aligned immediately adjacent to each other.

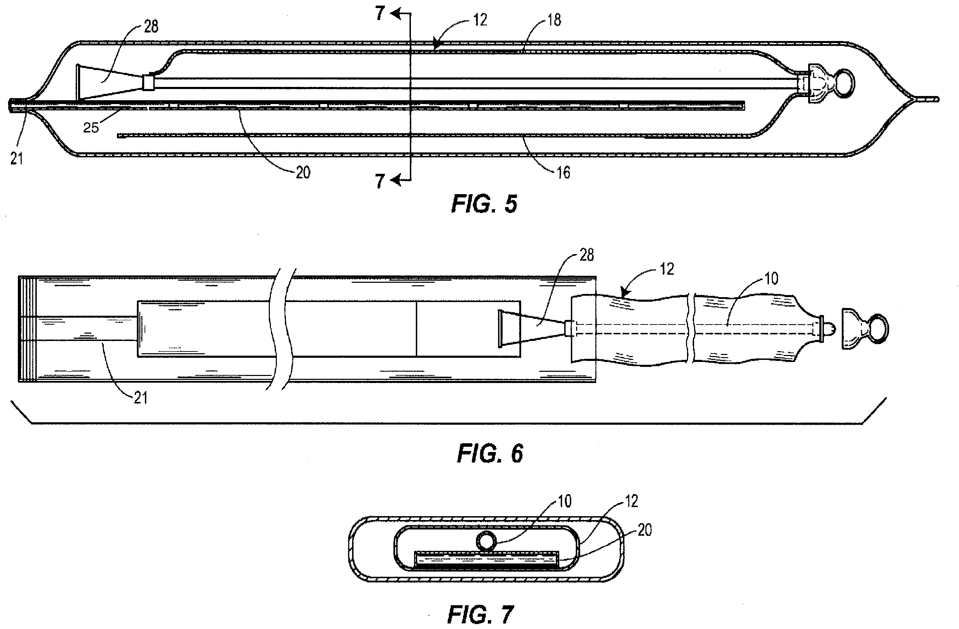

In a third embodiment, illustrated in Figs. 5-7, the liquid water impermeable, water vapor permeable membrane 20 forms a region or pocket 25 that is filled with liquid water (or contains or is comprised of one or more lengths of water-soaked fabric 22) and sealed or anchored to an external packaging layer by an anchoring tether 21, which may be an integral extension of the membrane 20, such that when the sleeved catheter 10 and sleeve 12 are pulled out of the package, the anchored membrane 20, which still contains some liquid water, is left behind in the package. In this embodiment, at least a portion of an end of the sleeve 12 is preferably sealed to the neck of the funnel 28 of the catheter 10. The pocket 25 defined by the liquid impermeable, water vapor permeable membrane 20 provides a location for the liquid water to be isolated from the catheter 10. As such, the membrane 20 prevents sufficient direct liquid contact with the hydrophilic coating on the catheter 10 to place the hydrophilic outer surface of the catheter 10 in an activated condition. As in the first and second embodiments, the liquid flow interfering element permits water vapor to permeate across the water vapor permeable wall of the membrane 20, eventually achieving sufficient vapor contact with the insertable length of the catheter 10 to hydrate its hydrophilic coating and render the catheter assembly ready to use.

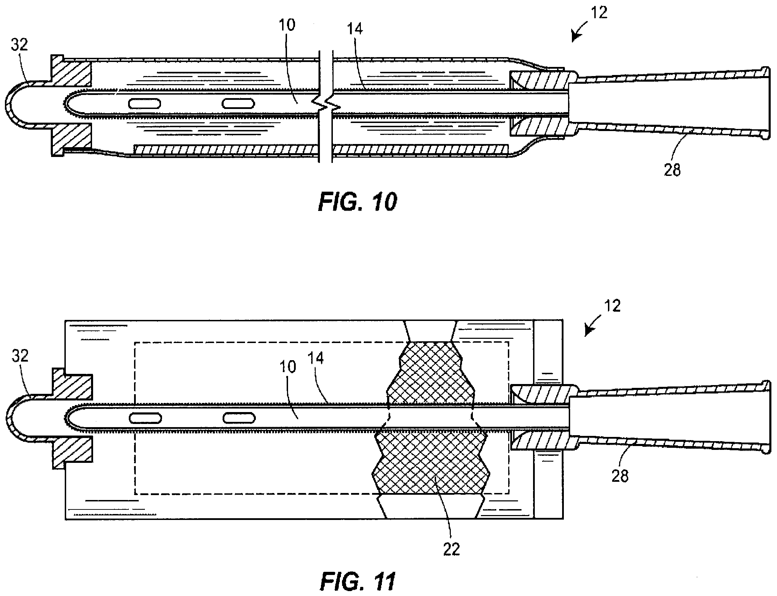

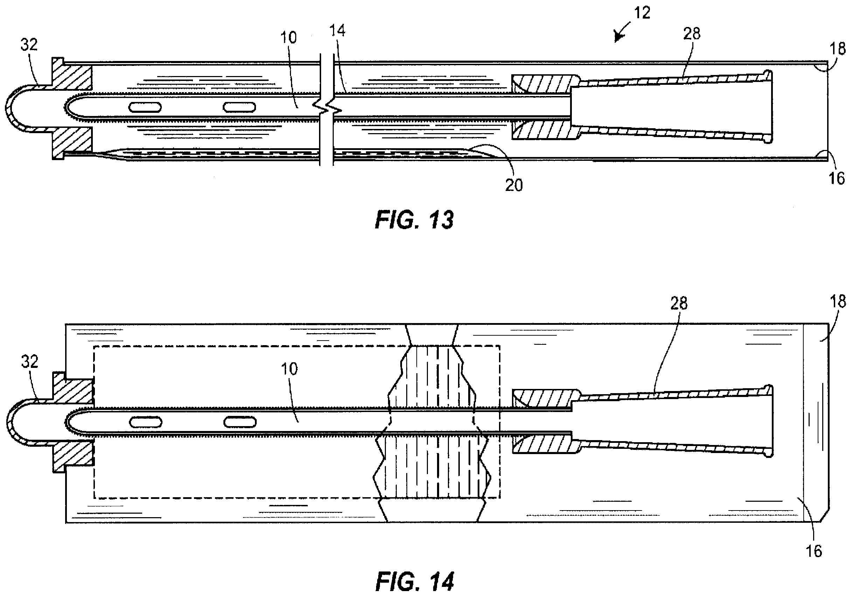

Alternately, the liquid water impermeable, water vapor permeable membrane 20 may be formed as an integral portion of the sleeve 12. As such, the membrane 20 is not left behind in a package surrounding the sleeve 12 upon removal of the sleeve 12 from the package. The sleeve may be sealed (with the seal preferably including an openable tear propagation line) beyond a funnel end of the catheter (as illustrated in the first embodiment of Figs. 1-2 and Figs. 8-9), sealed to an exterior of a neck an inlet of the funnel (according to a fourth embodiment illustrated in Figs. 10-12), or alternately, may extend beyond, but be open at a funnel end of the catheter (according to a fifth embodiment, as illustrated in Fig. 13-15).

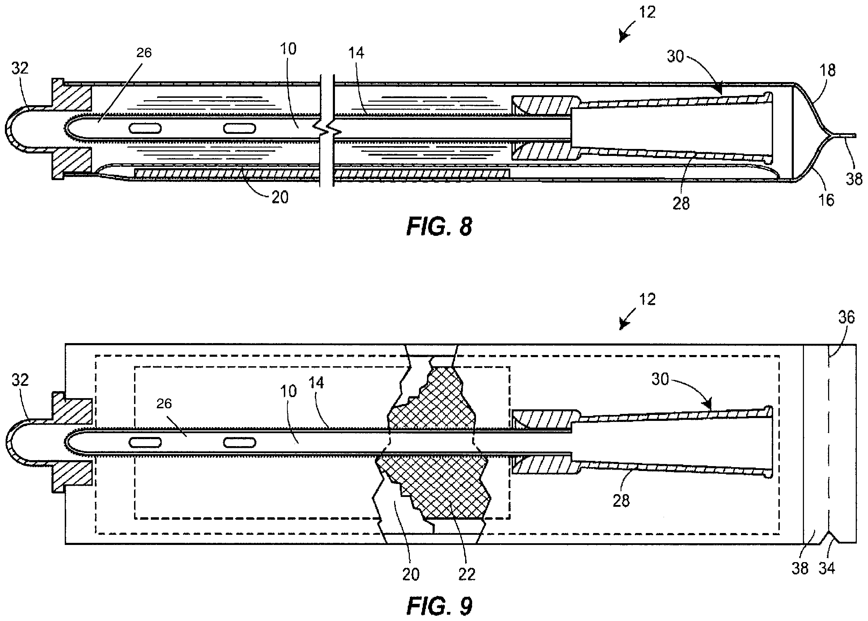

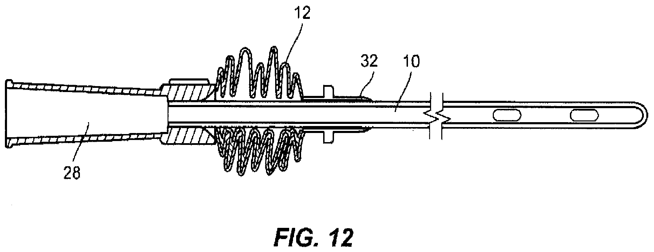

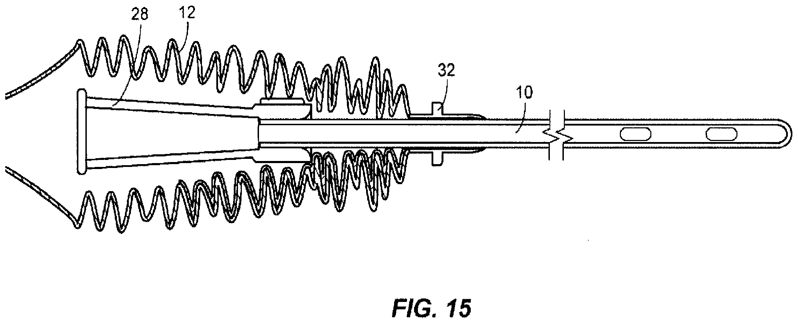

In any of the embodiments of the present disclosure, the catheter 10 has an eyed tip end 26 (Figs. 8 and 9), which is an insertion end of the catheter 10, and a funnel 28 provided at an opposite funnel end 30 of the catheter 10. The sleeve 12 is sealed to an introducer tip 32 and the catheter 10 is disposed in the sleeve 12 such that the insertion end of the catheter 10 is adjacent the introducer tip 32. The sleeve 12 is collapsible in a longitudinal direction, and is sufficiently thin so as to permit palpation of the catheter 10 through the walls of the sleeve 12, while isolating the user's fingers from contact with the activated hydrophilic coating. The user can manipulate the catheter 10 through the walls of the sleeve 12. Beginning with the insertion end, the user can advance the catheter 10 through the introducer tip 32. As the catheter 10 is advanced through the introducer tip 32, the sleeve continues to crumple, as illustrated in Figs. 12 and 15.

Turning back to the first embodiment, as illustrated in Figs. 8-9, the sleeve 12 is sealed beyond the funnel 28 to define the closed cavity, such that the entirety of the catheter 10, including the funnel end 30 of the catheter 10, is initially contained in the sleeve 12. The sealed end of the sleeve 12 adjacent the funnel end 30 of the catheter 10 may be provided with a tear-initiating notch 34 and, preferably, a weakened tear propagation line 36 across a sealed end zone 38 of the sleeve 12, as illustrated in Fig. 9. The funnel 28 has an outer diameter at some point along its length that is greater than an inner diameter of an opening in the introducer tip 32 through which the insertable length of the catheter 10 passes during insertion to the urethra.

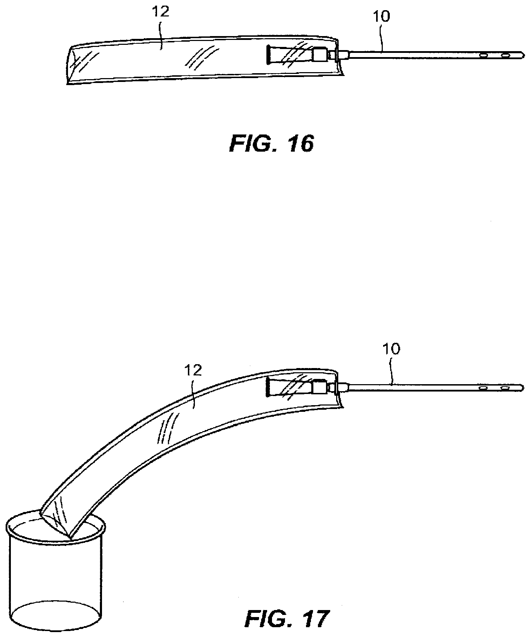

In embodiments where the sleeve 12 extends beyond the funnel 28, as illustrated in Figs. 8, 9, and 13-15 (i.e. where the sleeve 12 is not connected to the neck of the funnel 28 like in Figs. 10-12), once the insertable length of the catheter 10 is fully deployed through the introducer tip 32 and preferably, but not necessarily, prior to urine drainage, the user then slides the sleeve 12 back over and past the funnel 28, such that the sleeve 12 is fully extended (as illustrated in Fig. 16). The user tears the sealed end zone 38 using the tear- initiating notch 34 preferably by tearing along the tear propagation line 36, if provided, thereby opening the sealed end zone 38 of the sleeve 12. As illustrated in Fig. 17, the sleeve 12 can then be used to direct the flow of urine into a collection container, such as a bed pan or a collection bag, or into a toilet.

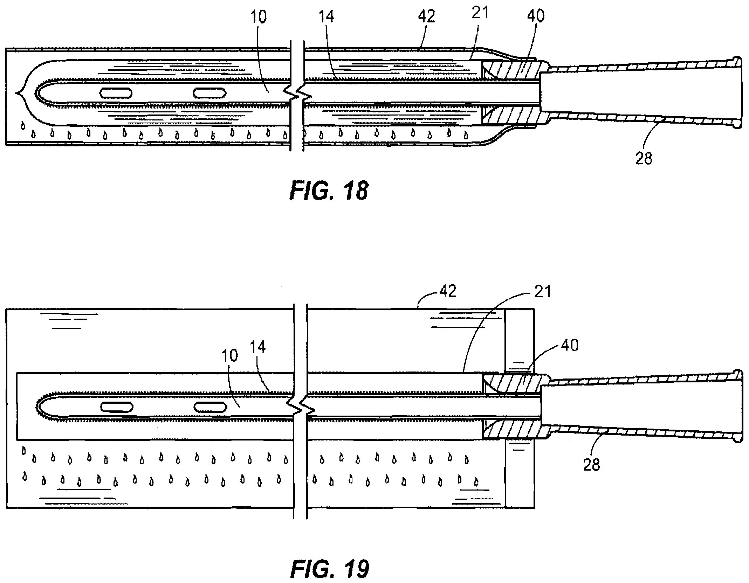

Turning now to Figs. 18 and 19, a sixth embodiment is illustrated in which the entire insertable portion of a catheter 10 having a hydrophilic coating 14 is enclosed in a sleevelike membrane 21 that forms an inner sleeve covering at least the insertable portion. The sleeve-like membrane 21 being a water vapor permeable polymer such as polyurethane or polyester, with a moisture vapor permeability greater than 300 g/ m²/day, 500 g/ m²/day, 1000 g/ m²/day, 2000 g/ m²/day or preferably greater than 3000 g/ m²/day. The sleeve-like membrane 21, which is liquid impermeable, is heat sealed to the collar 40 of a funnel 28 of the catheter 10. The sleeve-like membrane 21 is enclosed in a liquid and water vapor impermeable outer sleeve 42. The outer sleeve 42 is preferably made of polymeric films with a water permeability in the range of 0.1-10 g/ m²/day. In this embodiment, like the sleeve-like membrane 21, the outer sleeve 42 is sealed to the collar 40 of the funnel 28. Prior to sealing the outer sleeve 42 to the collar 40, water in liquid phase is introduced intermediate the exterior of the sleeve-like membrane 21 and the interior of the outer sleeve 42. Over time, an adequate amount of the liquid-phase water changes phase to water vapor, permeates the water vapor permeable sleeve-like membrane 21, and activates the hydrophilic coating 14.

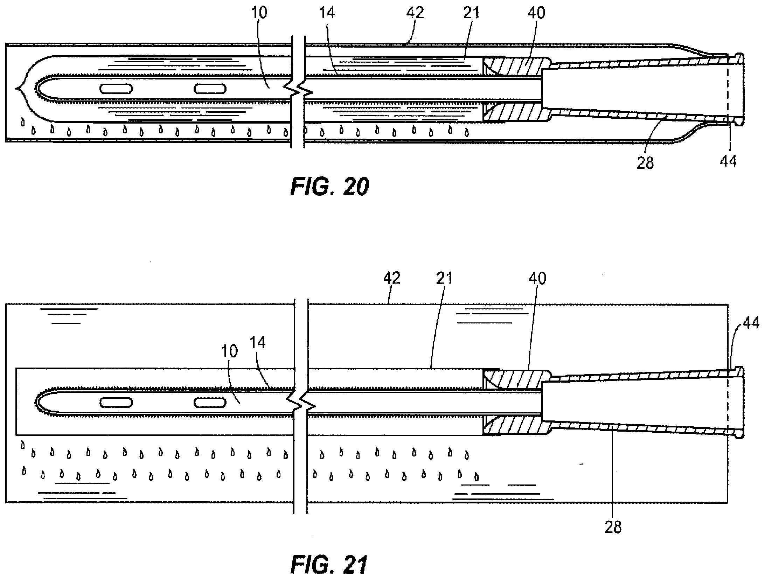

According to a seventh alternate embodiment, Figs. 20 and 21 illustrate a packaged catheter configuration in which, like the embodiment of Figs. 18-19, the entire insertable portion of a catheter 10 having a hydrophilic coating 14 enclosed in a water vapor permeable sleeve-like membrane or inner sleeve 21 that is heat sealed to the collar 40 of a funnel 28 of the catheter 10. While the sleeve-like membrane 21 of this embodiment is also enclosed in a liquid and water vapor impermeable outer sleeve 42, that outer sleeve 42 is sealed not to the collar 40, but rather, to the exterior of the funnel at an end 44 opposite the collar 40. Prior to sealing the outer sleeve 42 to the end 44 of the funnel 28 opposite the collar 40, water in liquid phase is introduced intermediate the exterior of the sleeve-like membrane 21 and the interior of the outer sleeve 42. Over time, an adequate amount of the liquid-phase water changes phase to water vapor, permeates the water vapor permeable sleeve-like membrane 21, and activates the hydrophilic coating 14.

According to an eighth embodiment, Figs. 22 and 23 illustrate a packaged catheter configuration in which, as in the sixth and seventh embodiments of Figs. 18-19 and 20-21, respectively, the entire insertable portion of a catheter 10 having a hydrophilic coating 14 enclosed in a water vapor permeable sleeve-like membrane or inner sleeve 21 that is heat sealed to the collar 40 of a funnel 28 of the catheter 10. In the eighth embodiment, while the entire sleeve is enclosed in a liquid and water vapor impermeable outer sleeve 42, that outer sleeve 42 is not sealed to the funnel 28 at all, but rather, to itself, at an outer sleeve end 46, completely enclosing the funnel 28. Prior to sealing the outer sleeve 42 at the outer sleeve end 46, water in liquid phase is introduced intermediate the exterior of the sleeve-like membrane 21 and the interior of the outer sleeve 42. A liquid impermeable barrier 52, such as a foil seal, may be sealed to the distal end of the funnel to prevent liquid from entering into the funnel and the inner lumen of catheter 10. Over time, an adequate amount of the liquid-phase water changes phase to water vapor, permeates the water vapor permeable sleeve-like membrane 21, and activates the hydrophilic coating 14. The outer sleeve end 46 preferably features a tear-initiating notch 48 leading to a tear track 50 that, when torn, permits excess liquid phase water remaining in the space intermediate the exterior of the sleeve-like membrane 21 and the interior of the outer sleeve 42 to be drained. The outer sleeve 42 may also be used in a fashion similar to the sleeve 12 of Fig. 17, to direct urine to an outside collection container, such as a bed pan or a collection bag, or into a toilet.

Fig. 24 illustrates an eyed inlet end 50 of the catheter 10 projecting through the sleeve-like membrane or inner sleeve 21 and the outer sleeve 42 of any of the embodiments of Figs. 18-23. The polyurethane or polyester material of which the walls of the sleeve-type membrane 21 are formed is preferably a film material that is sufficiently easy to penetrate that a user can urge the catheter 10 through walls of the sleeve-like membrane 21 by simply manipulating the catheter 10 from the exterior of the outer sleeve 42. The walls of the liquid and water vapor impermeable outer sleeve 42 are preferably more robust than the walls of the sleeve 12, such that the outer sleeve 42 may be provided with a weak point, a perforation, or a tear point in order to facilitate opening the outer sleeve 42 so as to expose the eyed inlet end 50 of the catheter 10. Alternately, it is recognized that an introducer tip 32 (similar to that illustrated, e.g., in Figs. 13-14) may be provided to expose the eyed inlet end 50 of the catheter 10 through both the outer sleeve 42 and the sleeve-like membrane 21, or at least through the outer sleeve 42 (with the ease of penetration of the film material of the sleeve 12 relied upon to expose the eyed inlet end 50 of the catheter 10 through the sleeve 12).

The following table provides coefficient of friction data for the hydrophilic coated catheters of the embodiments of the present disclosure after 6 weeks of storage with liquid contained intermediate the exterior of the sleeve 12, the membrane 20, or the sleeve-like membrane 21, and the interior of the outer sleeve 42, of the respective embodiments. These coefficients of friction were measured using Harland Friction tester model FTS5500. The test is set- up such that a 200g load is applied to a 127mm section of a fully hydrated catheter. The catheter is then pulled through 2 pieces of silicon rubber with 60 A Shore hardness at 10mm/s. The force required for the pulling the catheter over a distance of 80mm, out of a total length of 127mm, is measured using a universal tester equipped with 200N load cell. The CoF value is calculated from the ratio of applied to recorded loads when a steady state is reached. At least 5 measurements were carried out in each case and an average CoF value is reported.

| **Embodiment/sleeve** | **Figures** | **Average initial Coefficient of Friction (CoF)** | **Average ten min dry out CoF (*)** |
|---|---|---|---|
| | | Average of 2-10 samples | Average of 2-10 samples |
| First Embodiment | 1,2,8,9 | 0.017 | 0.042 |
| Second Embodiment | 3,4 | 0.025 | 0.047 |
| Third Embodiment | 5, 6, 7 | 0.029 | n/a |
| Fourth Embodiment | 10, 11 & 12 | 0.028 | 0.064 |
| Fifth Embodiment | 13, 14 & 15 | 0.023 | 0.045 |
| Sixth Embodiment | 18 & 19 | 0.02345 | 0.04215 |
| Seventh Embodiment | 20 & 21 | 0.0167 | 0.0288 |
| Eighth Embodiment | 22 & 23 | 0.02145 | 0.0275 |

| | | | |
|---|---|---|---|
| (*) ten minutes dry out refers to keeping the sample at 23°C and 50% RH for 10 minutes and re-measuring the CoF | | | |

While various embodiments have been described above, variations may be made thereto that fall within the scope of the appended claims.

## Claims

1. A dry-to-the touch, ready-to-use hydrophilic intermittent catheter assembly, comprising:
a catheter (10) having an insertable portion with a hydrophilic outer surface (14) in an activated condition, the catheter including a including a funnel (28) associated with a drainage end of the catheter (10) located opposite an insertion end thereof; **characterized in**:
a liquid and vapor impermeable sleeve (12) defining a closed cavity and covering at least the insertable portion of the catheter (10), the sleeve (12) being sealed at a seal zone (38) beyond the funnel (28) and the seal zone (38) provided with a weakened tear propagation line (36);
an introducer tip (32) adjacent to the insertion end of the insertable portion of the catheter (10) wherein the liquid and vapor impermeable sleeve (12) is sealed to the introducer tip (32) to define the closed cavity;
an amount of liquid disposed and contained within the closed cavity defined by the sleeve (12);
the amount of liquid being contained within a liquid flow interfering element (20, 23a) that interferes with liquid flow, the liquid flow interfering element contained within the closed cavity defined by the sleeve (12) and comprising a liquid impermeable, vapor permeable membrane (20) sealed to an inner surface of the sleeve (12) with the liquid being confined between the membrane (20) and the inner surface of the sleeve (12);
a vapor atmosphere contained within the cavity defined by the liquid and vapor impermeable sleeve (12) wherein the vapor atmosphere is produced by vapor donated from the amount of liquid contained within the liquid flow interfering element (20, 23a).;

2. The catheter assembly of claim 1 wherein the liquid impermeable, vapor permeable membrane (20) comprises thin films with water vapor permeability of greater 300 g/m2/day.

3. The catheter assembly of any one of claims 1 and 2 wherein the liquid impermeable, vapor permeable membrane (20) is a bacteria barrier.

4. The catheter assembly of any one of claims 1 - 3 wherein the liquid flow interfering element further includes a wicking element (22) between the membrane (20) and the inner surface of the sleeve (12) to evenly disperse the liquid confined between the membrane (20) and the inner surface of the sleeve (12).

5. The catheter assembly of claim 1 including a notch (34) associated with the seal zone (38) of the sleeve (12) beyond the funnel (28) for opening the sleeve (12) to permit urine to drain from the sleeve (12) after the sleeve is opened.

## Patentansprüche

1. Gebrauchsfertige hydrophile intermittierende Katheteranordnung mit Trockengriff, umfassend:
einen Katheter (10) mit einem einführbaren Abschnitt mit einer hydrophilen Außenfläche (14) in einem aktivierten Zustand, wobei der Katheter einen Trichter (28) beinhaltet, der einem Drainageende des Katheters (10) zugeordnet ist, das gegenüber einem Einfügeende davon angeordnet ist; **gekennzeichnet durch**:
eine flüssigkeits- und dampfundurchlässige Hülle (12), die einen geschlossenen Hohlraum definiert und zumindest den einführbaren Abschnitt des Katheters (10) bedeckt, wobei die Hülle (12) an einer Dichtungszone (38) jenseits des Trichters (28) und der Dichtungszone (38), die mit einer geschwächten Weiterreißlinie (36) versehen ist, abgedichtet ist;
eine an das Einfügeende des einführbaren Abschnitts des Katheters (10) angrenzende Einführungsspitze (32), wobei die flüssigkeits- und dampfundurchlässige Hülle (12) an der Einführungsspitze (32) abgedichtet ist, um den geschlossenen Hohlraum zu definieren;
eine Flüssigkeitsmenge, die in dem durch die Hülle (12) definierten geschlossenen Hohlraum angeordnet und enthalten ist;
wobei die Flüssigkeitsmenge in einem den Flüssigkeitsstrom störenden Element (20, 23a) enthalten ist, das den Flüssigkeitsstrom stört, wobei das den Flüssigkeitsstrom störenden Element in dem durch die Hülle (12) definierten geschlossenen Hohlraum enthalten ist und eine flüssigkeitsundurchlässige, dampfdurchlässige Membran (20) umfasst, die an einer Innenfläche der Hülle (12) abgedichtet ist, wobei die Flüssigkeit zwischen der Membran (20) und der Innenfläche der Hülle (12) eingeschlossen ist;
eine Dampfatmosphäre, die in dem Hohlraum enthalten ist, der durch die flüssigkeits- und dampfundurchlässige Hülle (12) definiert ist, wobei die Dampfatmosphäre durch Dampf erzeugt wird, der von der Flüssigkeitsmenge abgegeben wird, die in dem den Flüssigkeitsstrom störenden Element (20, 23a) enthalten ist.

2. Katheteranordnung nach Anspruch 1, wobei die flüssigkeitsundurchlässige, dampfdurchlässige Membran (20) dünne Filme mit einer Wasserdampfdurchlässigkeit von mehr als 300 g/m2/Tag umfasst.

3. Katheteranordnung nach einem der Ansprüche 1 und 2, wobei die flüssigkeitsundurchlässige, dampfdurchlässige Membran (20) eine Bakterienbarriere ist.

4. Katheteranordnung nach einem der Ansprüche 1-3, wobei das den Flüssigkeitsstrom störende Element ferner ein Dochtelement (22) zwischen der Membran (20) und der Innenfläche der Hülle (12) beinhaltet, um die eingeschlossene Flüssigkeit gleichmäßig zwischen der Membran (20) und der Innenfläche der Hülle (12) zu verteilen.

5. Katheteranordnung nach Anspruch 1, mit einer Kerbe (34), die der Dichtungszone (38) der Hülle (12) jenseits des Trichters (28) zugeordnet ist, um die Hülle (12) zu öffnen, damit Urin aus der Hülle (12) abfließen kann, nachdem die Hülle geöffnet wurde.

## Revendications

1. Ensemble de cathéter intermittent hydrophile, sec au toucher et prêt à l'emploi, comprenant :
un cathéter (10) ayant une partie insérable avec une surface externe hydrophile (14) dans un état activé, le cathéter comprenant un entonnoir (28) associé à une extrémité de drainage du cathéter (10) située à l'opposé d'une extrémité d'insertion de celui-ci ; **caractérisé par** :
un manchon imperméable aux liquides et à la vapeur (12) définissant une cavité fermée et recouvrant au moins la partie insérable du cathéter (10), le manchon (12) étant scellé au niveau d'une zone d'étanchéité (38) au-delà de l'entonnoir (28) et de la zone d'étanchéité (38) munie d'une ligne de propagation de déchirure affaiblie (36) ;
une pointe d'introduction (32) adjacente à l'extrémité d'insertion de la partie insérable du cathéter (10) dans lequel le manchon imperméable aux liquides et à la vapeur (12) est scellé à la pointe d'introduction (32) pour définir la cavité fermée ;
une quantité de liquide disposée et contenue dans la cavité fermée définie par le manchon (12) ;
la quantité de liquide étant contenue dans un élément interférant avec l'écoulement de liquide (20, 23a) qui interfère avec l'écoulement de liquide, l'élément interférant avec l'écoulement de liquide contenu dans la cavité fermée étant définie par le manchon (12) et comprenant une membrane imperméable aux liquides et perméable à la vapeur (20) scellée à une surface intérieure du manchon (12), le liquide étant confiné entre la membrane (20) et la surface intérieure du manchon (12) ;
une atmosphère de vapeur contenue dans la cavité définie par le manchon imperméable aux liquides et à la vapeur (12) dans lequel l'atmosphère de vapeur est produite par la vapeur donnée à partir de la quantité de liquide contenue dans l'élément interférant avec l'écoulement de liquide (20, 23a). ;

2. Ensemble de cathéter selon la revendication 1, dans lequel la membrane imperméable aux liquides et perméable à la vapeur (20) comprend des films minces avec une perméabilité à la vapeur d'eau supérieure à 300 g/m2/jour.

3. Ensemble de cathéter selon l'une quelconque des revendications 1 et 2, dans lequel la membrane imperméable aux liquides et perméable à la vapeur (20) est une barrière bactérienne.

4. Ensemble de cathéter selon l'une quelconque des revendications 1 à 3, dans lequel l'élément interférant avec l'écoulement de liquide comprend en outre un élément à effet de mèche (22) entre la membrane (20) et la surface intérieure du manchon (12) pour disperser uniformément le liquide confiné entre la membrane (20) et la surface intérieure du manchon (12) .

5. Ensemble de cathéter selon la revendication 1, comprenant une encoche (34) associée à la zone d'étanchéité (38) du manchon (12) au-delà de l'entonnoir (28) pour ouvrir le manchon (12) afin de permettre à l'urine de s'écouler du manchon (12) après l'ouverture du manchon.
